# EUROPEAN PATENT SPECIFICATION

(11) **EP 3 301 186 B1**
(45) Date of publication and mention of the grant of the patent: **27.01.2021**
(21) Application number: 16732356.7
(22) Date of filing: 02.05.2016
(51) Int. Cl.: C12Q 1/6886

(54) **METHOD, SEQUENCES, COMPOSITIONS AND KIT FOR DETECTION OF MUTATIONEN IN THE PROMOTER OF THE GENE HTERT**
METHODEN, SEQUENZEN, ZUSAMMENSETZUNGEN UND KIT ZUM NACHWEIS VON MUTATIONEN IM PROMOTER DES HTERT GENS
MÉTHODE, SÉQUENCES, COMPOSITIONS ET TROUSSE POUR LA DÉTECTION DE MUTATIONS DANS LE PROMOTEUR DU GÈNE HTERT

(30) Priority: 30.04.2015 PT 2015108419
(43) Date of publication of application: 04.04.2018
(73) Proprietor: IPATIMUP ( Instituto de Patologia e Imunologia Molecular da Universidade do Porto, 4200-465 Porto (PT)
(72) Inventor: SOARES DIAS FERREIRA, Ana Paula, 4000-292 Porto (PT); MARQUES PRAZERES, Hugo João, 3200-343 Serpins (PT); ALVES SALGADO, Catarina Miguel, 4510-555 Fânzeres (PT); MONTEIRO BATISTA, Rui Pedro, 5000-105 Vila Real (PT); RICO DE OLIVEIRA VINAGRE, João Pedro, 3880-272 Ovar (PT)
(74) Representative: Ferreira Pinto, Francisca
(86) International application number: PCT/PT2016/050007
(87) International publication number: WO 2016/175672

(56) References cited:
- WO-A1-2015/049063
- YVES ALLORY ET AL: "Telomerase Reverse Transcriptase Promoter Mutations in Bladder Cancer: High Frequency Across Stages, Detection in Urine, and Lack of Association with Outcome", EUROPEAN UROLOGY, vol. 65, no. 2, 1 February 2014 (2014-02-01), pages 360-366, XP055305481, NL ISSN: 0302-2838, DOI: 10.1016/j.eururo.2013.08.052
- CHRISTIAN KOELSCHE ET AL: "TERT promoter hotspot mutations are recurrent in myxoid liposarcomas but rare in other soft tissue sarcoma entities", JOURNAL OF EXPERIMENTAL & CLINICAL CANCER RESEARCH, BIOMED CENTRAL LTD, LONDON UK, vol. 33, no. 1, 11 April 2014 (2014-04-11) , page 33, XP021182412, ISSN: 1756-9966, DOI: 10.1186/1756-9966-33-33
- KUN WANG ET AL: "TERT promoter mutations are associated with distant metastases in upper tract urothelial carcinomas and serve as urinary biomarkers detected by a sensitive castPCR", ONCOTARGET, vol. 5, no. 23, 9 December 2014 (2014-12-09), pages 12428-12439, XP055305432, DOI: 10.18632/oncotarget.2660

## Description

The present invention refers to a method for the detection of the c.-124 C>T and c.-146 C>T mutations in Htert gene promoter. The referred method uses a reaction composition that comprises primers for amplification and probes for genotyping.

Another aspect of this invention refers the primers and probes used in performing the aforementioned method with sequences, identified as SEQ ID NO: 1 to SEQ ID NO: 6, that display high specificity for these mutations, as well as compositions that contain them.

The present invention further refers to a kit comprising the above mentioned compositions for detecting mutations c.-124 C>T and c.-146 C>T mutations in Htert gene promoter by conducting the present method invention.

The method, gene sequences, compositions and kit of the present invention can be advantageously used for detecting trace amounts of c.-124 C>T and c.-146 C>T mutations, present in biological samples due to its high sensitivity and specificity for such mutations.

The present invention can therefore be applied in early detection, identification, detection of recurrence or prediction and monitoring of diseases associated with those mutations, such as bladder carcinomas, thyroid carcinomas, squamous cell carcinoma, basal cell carcinomas, skin cancer, central nervous system cancers and hepatocellular carcinoma, among others and eventually provide the basis for appropriate treatment setting.

Thus, the present invention falls within the technical field of medicine, pharmaceutics, molecular biology, biochemistry, and human related genetics.

### DESCRIPTION

### METHOD, SEQUENCES, COMPOSITIONS AND KIT FOR DETECTION OF CHANGES IN THE PROMOTER OF THE GENE Htert

### Technical field of the invention

The present invention refers to a method for the ultrasensitive detection of c.-124 C>T and c.-146 C>T mutations in Htert gene promoter, the reaction compositions comprising primers for amplification and probes for genotyping, the sequences of referred as SEQ ID NO: 1 to SEQ ID NO: 6, that were designed to display a high specificity for these mutations, as well as, the kit comprising such compositions for performing the referred method.

The present invention can advantageously be used to detect trace amounts of such c.-124C>T and c.-146C>T mutations present in biological samples and *in vitro* due to the high sensitivity of the method and specificity of gene sequences created, and allowing thereby the early detection, recurrence identification or prediction and monitoring of diseases associated with those mutations, such as bladder carcinomas, thyroid carcinomas, squamous cell carcinoma, basal cell carcinomas, skin cancer, central nervous system cancers and hepatocellular carcinoma, among others and eventually provide the basis for appropriate treatment setting.
Thus, the present invention falls within the technical field of medicine, pharmaceutics, molecular biology, biochemistry, and human related genetics.

### State of the art

Somatic mutations that occur during DNA replication that precede a mitotic division, are at the origin of certain human disorders, particularly certain types of cancers. The presence of the enzyme telomerase was observed in a high percentage, about 75-80%, of tumor cell lines. Recently, the applicants have identified mutations in the telomerase gene promoter encoding this enzyme, the TERT gene, particularly, the mutations at positions c.-124C>T and c.-146C>T (from the initial ATG). These mutations have been associated with several malignancies, such as central nervous system cancers (43-51%), bladder carcinomas (59-66%), hepatocellular carcinomas (59%), thyroid carcinomas (10%), skin cancers (melanoma 29% -73%, 73% basal cell carcinoma (BCC) and squamous cell carcinomas (SCC) 45%) and gastrointestinal stromal tumours (GISTs) (4%) [1-7].

The fact that mutations in telomerase promoter are present in a relatively high frequency in certain cancers makes them potential biomarkers for early detection, diagnosis, prognosis, recurrence detection or prediction/monitoring of therapy response of tumors, such as, central nervous system tumours, bladder carcinomas, hepatocellular carcinomas, thyroid carcinomas, and skin cancers, among others. For example, in bladder cancer, somatic mutations in positions c.-124C>T and c.-146C>T (from the ATG) of the telomerase gene have a prevalence that reaches 85%, and, along with the mutations R248C and S249C in the fibroblast growth receptor 3 (FGFR3) gene [9], are the most reliable biomarkers for bladder cancer.

However, for the *in vitro* use of biological sample fluids (e.g. urine, plasma, cerebrospinal fluid, aspiration cytology, among others), the mutation detection method in positions c.-124 C>T and c.-146C>T (from the ATG) of the telomerase gene must have high analytical sensitivity in order to detect trace amounts of the mutated alleles, even when they are "diluted" in a sample with greater abundance of alleles "wild-type".

The document WO2015153808 discloses methods and compositions useful in the detection of some diseases, in particular cancer, more specifically, thyroid cancer, related to mutations at positions c.-124C>T and c.-146C>T of the TERT gene. However, the nucleotide sequences disclosed in that document are used for detection of such mutations by direct amplification methods, sequence dependent, and have limited sensitivity when the presence of normal sequences is relatively high compared to the mutant sequences. On the other hand, they are susceptible to the occurrence of non-specific amplification, so that, they do not exhibit high specificity for this type of mutation, which causes, in its general method not to be sufficiently sensitive and specific.

The document WO2014160834 discloses methods and compositions useful in the detection and treatment of different types of thyroid cancer, related to mutations at positions c.-124 C> T and c.-146 C>T TERT gene. However, in this document the disclosed nucleotide sequences did not show high sensitivity since the presence of normal sequences is very abundant when compared to the mutant sequences, and the proposed method is based on DNA sequencing using "BigDye terminator v3.1" sequencing ready kit and in a Applied Biosystems ABI PRISM equipment 3730, which has a limited sensitivity to detect rare alleles in the reaction. Moreover, the presence of the mutation must be confirmed by sequencing in both directions, sense and antisense, which makes the method impractical and laborious and apply only to thyroid cancers.

Yves Allory et al: "Telomerase Reverse Transcriptase Promoter Mutations in Bladder Cancer: High Frequency Across States, Detection in Urine, and Lack of Association with Outcome", EUROPEAN UROLOGY, vol. 65, no.2, 1 February 2014 (2014-02-01), pages 360-366, describes a SNaPshot assay to detect mutants referred in the present invention. The fragment comprising the mutations is amplified with a primer pair and the mutations are detected each with a probe, which are used in a single nucleotide extension reaction. In the present invention other primers are used and the probes are allele specific without single nucleotide extension.

Christian Koelsche et al: "Tert promoter hotspot mutations are recurrent in myxoid liposarcomas but rare in other soft tissue sarcoma entities",JOURNAL OF EXPERIMENTAL & CLINICAL CANCER RESEARCH BIOMED CENTRAL LTD, LONDON UK, vol.33, no.1, 11 April 2014 (2014-04-11), page 33, describes a method using PCR and direct sequencing for detecting the mutations. The primers and probes used in the method described herein are different to those defined in the present invention.

Kun Wang et al: "Tert promoter mutations are associated with distant metastases in upper tract urothelial carcinomas and serve as urinary biomarkers detected by a sensitive castPCR", Oncotarget, vol. 5, no.23, 9 December 2014 (2014-12-09), pages 12428-12439, describes Sanger sequencing to detect the mutations described in the present invention. In particular this reference describes a very specific and sensitive castPCR method to detect C228T, which uses allele specific primers and blockers and a detection probe.

WO2015/049063, in the name of Deutsches Krebsforschung, (2015-04-09), describes a widely used method for the detection of the two mutations, which comprises an amplification step followed by a sequencing step. However the primer/probe combinations used in D4 are different to those of the present invention.

The methods described in the art to discriminate mutations at positions c.-124C>T and c.-146C>T of the TERT gene that comprise sequencing or single base extension have the disadvantage that after amplification, a second reaction (sequencing or SNaPshot or single base extension) needs to be performed, thus posing the problem of high risk of contamination derived from manipulation of amplification products when the discrimination is not performed in a single reaction.

Furthermore, in sequencing or single base extension, equimolar amounts of dideoxynucleotides are used and there is no bias towards the detection of the mutant allele, with the disadvantage that reaction resources are consumed by extensions of the wild-type alleles and posing the problem of lack of a reaction bias towards the detection of the mutant alleles.

To achieve biased amplification of the mutant alleles, methods in the art are based either in the use of blocker of the wild-type sequence or in sequence-specific primers for amplification of the mutant allele with allele specific PCR primers.

Methods that use PCR blockers to supress the amplification of the wild-type allele, known as Cast PCR, favour the use of reaction resources towards the amplification of the mutant alleles. However, because the wild-type allele amplification is supressed in the reaction, there is no internal control in the reaction for amplification of the specific target sequence and in this condition, the lack of amplification of mutant alleles cannot be distinguished from inhibition of the amplification of the target sequence due for example to contaminants or impurities. Usually when performing Cast PCR, a reaction with probes for detecting the wild-type allele needs to be performed in parallel, in a separate reaction using the same sample, so as to certify that sample is adequate for amplification. However this does not allow a true endogenous control to be present on the mutant-detection reaction and endogenous control and target sequences cannot be detected within a single reaction.

Methods based on allele-specific PCR use sequence-specific primers so that the 3' nucleotide of the primer is complementary to the mutant nucleotide in the target sequence, thus inhibiting the chance that DNA Polymerases extend the primers with 3' overhangs. However it is known that even in the lack of 3' complementarity DNA Polymerases may extend mismatched primers and, since the primer becomes integrated in a sequence, which is then targeted in a new amplification round, this can generate an exponential unspecific amplification.

Other methods, such as Real-time PCR for allelic discrimination, comprise amplification of a target sequence encompassing a specific mutant site followed by hybridization of the produced amplimers with wild-type and mutant sequence specific labelled probes, that are degraded in the extension phase by 3'-5' DNA Polymerase exonuclease activity to produce fluorescence. Equimolar concentrations of probes for wild-type and mutant alleles are employed and this has the disadvantage that no bias for use of reaction resources in favour of the detection of the mutant allele exists.

Also, no method to discriminate mutations at positions c.-124C>T and c.-146C>T of the TERT gene known in the art can be calibrated for a desired analytical sensitivity/detection limit and this can't be differentially set for one or other allele on the basis of the reaction composition. This is a disadvantage of current methods since it is known that analytical sensitivity doesn't necessarily follow clinical sensitivity and detection of very low levels of a biomarker may occur in a subclinical situation and not necessarily translate into a clinical expression. A method in which analytical detection limits could be calibrate for clinical expression would have higher Positive Predictive Value and higher clinical specificity, as few false positives would be generated.

Thus, it is necessary to develop a method with sufficient sensitivity and specificity that allows the detection of such mutations in biological samples even in conditions in which the mutated alleles are present in very low amounts.

The present invention proposes an ultrasensitive method that allows the detection of mutations c.-124 C>T and c.-146 C>T in Htert gene promoter, by designing primers and probes whose sequences are specific for this purpose.

### Summary of the Invention

The present invention refers to the primer set,probe sequences, compositions, kits and methods as defined in the claims 1 to 14. These primers and DNA probes, because they have high specificity in the detection of such mutations allow its use in a method for high sensitivity detection of these mutations.

The compositions of the present invention are applicable to the detection of mutations c.-124 C>T and c.-146 C>T in Htert gene promoter, in combinations that allows the detection of both the endogenous control wild-type allele and the mutant alleles to be detected in the same reaction, at differential analytical sensitivities, with bias towards the detection of the mutant alleles and with detection limits that can be calibrated by manipulating the reaction composition that may be pre-prepared or may be prepared at the time of performing the detection method of the invention.
In these compositions, the amount of DNA in the test sample can be quite low, given the high specificity of the primers and probes of the present invention designed for that purpose.

The kit of the present invention has the advantage of developing ultrasensitive method of *in vitro* detection of mutations c.-124 C>T and c.-146 C>T in Htert gene promoter, a quick and reliable manner, by adding a sample human DNA test for the detection of said mutation.

The method of the present invention may advantageously be used to detect trace quantities of such mutations c.-124 C>T and c.-146 C>T present in biological samples due to its high sensitivity conferred by the genetic sequence designed for this purpose and which have a high specificity for such mutations.
Thus, the present invention can be applied in early detection, identification, recurrence or prediction detection and monitoring of diseases associated with such mutations, such as bladder carcinomas, thyroid carcinomas, skin cancers, central nervous system cancers and hepatocellular carcinoma among other and eventually provide the basis for the appropriate treatment setting.

### Description of the invention

The present invention refers to a method for *in vitro* detection of mutations c.-124 C>T and c.-146 C>T M in Htert gene promoter. The referred reaction method uses compositions comprising primers for amplification and probes for genotyping, whose sequences (SEQ ID NO: 1 to SEQ ID NO:6) are specific for these mutations.
Thus, primer sequences are created for amplification and probes for genotyping in order to build the basis for said amplification reaction, eg. a PCR assay Real - Time (RT-PCR) or Digital Time PCR. 6 sequences were designed for the primers and probes, as shown in the sequence list. This includes the sequences for the following purposes:
a. Detection of the mutation located at -124 bases upstream of the ATG of the *hTERT* gene
   - SEQ ID NO: 1: Initiator F
   - SEQ ID NO: 2: Initiator R
   - SEQ ID NO: 3: Probe -124C
   - SEQ ID NO: 4: Probe- 124T
b. Detection of the mutation located at -146 bases upstream of the ATG of the *hTERT* gene
   - SEQ ID NO: 1: Initiator F
   - SEQ ID NO: 2: Initiator R
   - SEQ ID NO: 5: Probe -146C
   - SEQ ID NO: 6: Probe- 146T

The above sequences can be prepared by the nucleotide synthesis method using the solid phase phosphoramidite nucleosides as described in the publication: Beaucage, SL; Iyer, RP (1992). "Advances in the Synthesis of Oligonucleotides by the Phosphoramidite Approach". Tetrahedron 48 (12): 2223. The synthesis can be performed on columns with solid support (controlled pore glass or polystyrene) functionalized with the first base of the 3 'end of each oligonucleotide. The preparation of each oligonucleotide follows after a number of synthesis cycles, each consisting of chemical reactions, typically four chemical reactions: i) release (detritylation), ii) coupling, iii) protection and iv) oxidation. In each cycle are added at the 5 'terminus of the growing chain, nucleotide residues corresponding to the desired sequence.

These can be further modified at the 5 'and 3' sequences of the probes by the addition of corresponded fluorophores such as FAM, Yakima Yellow, quencher (TAMRA), etc. known to experts, for the purpose of amplification and hybridization reactions in multiplex PCR. Thus, suitable fluorophores in the present invention are, for example compounds known to Alexa Fluor FAM, TET, JOE, VIC, HEX, Cy3, ATTO 550, TAMRA, ROX, Cy5, Cy5.5.

The nucleotides refer to nucleotides of natural or synthetic origin, with the hybridization capacity by base-pairing with complementary nucleotides, and may include, without limitation, DNA, RNA, and nucleotide analogues (e.g. nucleic acids with closed conformation, known as "locked nucleic acids" - LNA) nucleotides or without inter-nucleotide phosphodiester type linkages (e.g. peptide nucleic acid - PNA) and nucleic acids with tiodiester bonds, or the like for the same purpose.

Compositions for amplification and DNA hybridization (PCR compositions) were prepared by adding reactant solutions to PCR, available on the market, with different nucleotide sequences with SEQ ID NO: 1 to SEQ ID NO: 6 in different concentrations, and bi-distilled water and deionized water-bi.

The reaction solutions for PCR used vary with the desired type of amplification reaction. Examples of suitable reactant solutions for PCR for the embodiment of the present invention are for example the solution "TaqMan® Universal PCR Master Mix" Thermofischer Scientific company or similar solutions for the purpose of DNA amplification and hybridization.

The concentrations of probes refer to the molar concentration of the solution, corresponding to the number of moles of each probe per volume of solution, wherein nanoMolar (nM) corresponds to 1x10 ⁻⁹ mole per liter.

Compositions for PCR were prepared comprising various combinations of different concentrations of the probes with sequences SEQ ID NO: 3 to SEQ ID NO: 6. As mentioned previously, probes whose sequences are defined by SEQ ID NO: 3 and SEQ ID NO: 4 were designed for the specific detection of c.-124 C>T mutation in the Htert gene promoter while the probes SEQ ID NO: 5 and SEQ ID NO: 6 were designed for the specific detection of c.-146 C>T mutation in the Htert gene promoter.

Thus, the compositions of the invention for PCR may comprise two sets of probes mentioned above, i.e., the compositions of the PCR of the invention comprise all the probes with SEQ ID NO: 3 to SEQ ID NO: 6 (c.-124 + c.-146 complete set).

Both sets of probes are present in the compositions for PCR, then these compositions are specific for the detection of both c.-124 C>T and c.-146 C>T mutations.

The concentration of nucleotide probes of the present invention in the solutions is adjusted to the PCR, in order to promote the sensitivity of the method of the invention the detection of such mutations. Preferably, the final concentration of each probe is adjusted to values between 400 and 1600nM, more preferably with a final concentration values of 800 to 1600nM and in a further preferred embodiment of the invention the values of the final concentration of each probe is approximately 1600nM.

Thus, it is possible to obtain compositions for PCR comprising different combinations of final concentration values of each of the probes defined by SEQ ID NO: 3 and SEQ ID NO: 6.

Thus, in a preferred form, the compositions for PCR comprising the following concentrations of the probes with SEQ ID NO: 3 to SEQ ID NO: 6:
i. of 250nM to 500nM of SEQ ID NO: 3, 400nm to 1600nM of SEQ ID NO: 4 of 250nM to 500nM of SEQ ID NO: 5 and 400nm to 1600nM of SEQ ID NO: 6,
   or
ii. of 250nM to 500nM of SEQ ID NO: 3, of 800nm to 1600nM of SEQ ID NO: 4 of 250nM to 500nM of SEQ ID NO: 5 and 800nm to 1600nM of SEQ ID NO: 6.

The compositions of the reaction mixtures for the detection of mutations c.-124 C>T and c.-146 C>T in the Htert gene promoter were prepared by adding DNA from the biological sample to be tested to the compositions mentioned above for PCR.

The test DNA can be obtained from tissue, urine, circulating tumour DNA, germline or other biological sources using standard methods known in the art such as those commercialized by Qiagen QIAamp® company or similar methods. The amount of DNA to be tested can be present in the composition to detect mutations c.-124 C>T and c.-146 C>T in Htert gene promoter, at concentrations below 500 ng, less than 100ng, 50ng and the upper to 1 ng.

The referred adjust in the concentration of the probes used is intended to boost the analytical sensitivity of the method of the present invention, demonstrated by a minimum number of mutant alleles (DNA MUT) detected relative to the number of alleles "wild-type" (DNA WT). It is assumed that the increase in the analytical sensitivity results from increased concentrations of the probes used in the reaction by favouring amplification of the mutant allele.

Simultaneously, it reduced the efficacy of hybridization and amplification of the allele "wild-type" in order to keep as much as possible the balance of the reaction in favour of amplification of the mutant allele.

Thus, it was possible to develop a method in which various combinations of different concentrations of the probes used give rise to conditions that boost the analytical sensitivity and can detect very low levels of mutant alleles.

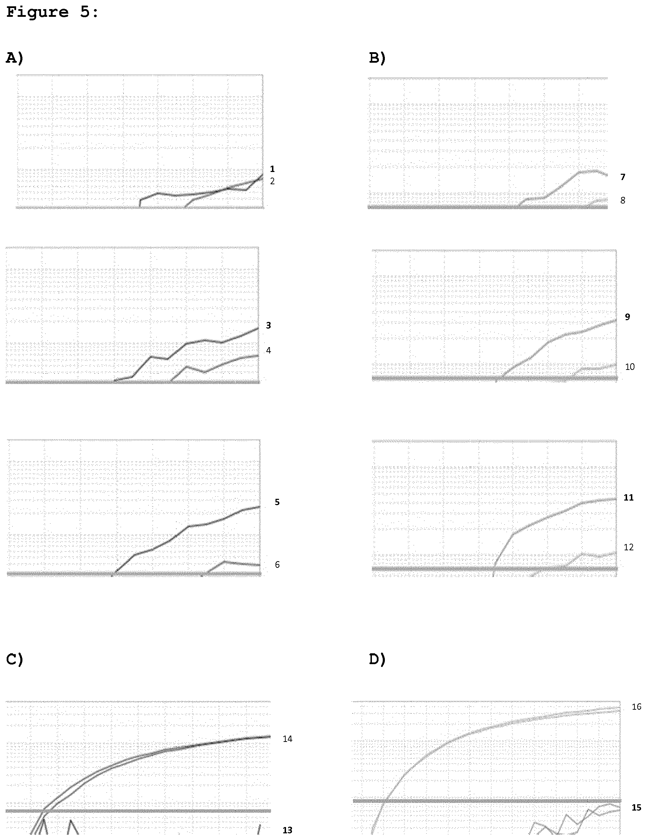

Up to certain levels, the increase in the concentration of mutated probe does not result in nonspecific detection of allele "wild-type" as is evidenced by the absence of signal from probes -124T and -146T when using only DNA WT-like sample (Fig. 5 - panels C and D). Thus, based on the manipulation of the probe concentrations used, it is possible to calibrate the sensitivity of the analytical method while its specificity is retained.

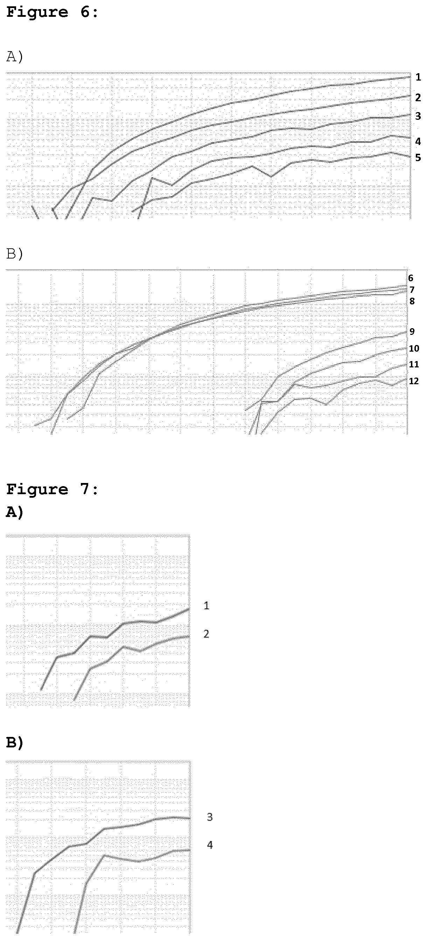

In reflexion of this increased efficiency in the detection of mutated alleles -124T and -146T, is the significantly increase of the analytical sensitivity of the method, passing to minimum detection values of 1.56% for -124T allele and 0.78% for -146T allele (Fig. 6A and B, respectively).

Thus, the method of the present invention comprises the steps of: i) preparing a composition for detecting mutations c.-124 C>T and c.-146 C>T M in Htert gene promoter, such as described above, ii) promotion of DNA extracted from a biological sample to be tested for the mutation, a composition for PCR amplification and hybridization, also as described above, iii) amplification and hybridization of the sample composition DNA for detecting mutations c.-124 C>T and c.-146 C>T in the Htert gene promoter by PCR reaction, real time PCR, RT-PCR or digital PCR or even multiplex PCR reaction, and iv) analysing the amplification curves thus obtained.

The presence of c.-124 mutated C>T and c.-146 C>T in the Htert gene promoter of test sample is analysed for the existence of an exponential growth of a fluorescence signal, which corresponds to the presence of at least one of the referred mutations.
The method of the present invention is useful in detection of trace amounts of c.-124 C>T and c.-146 C>T mutations in several biological fluids, e.g., urine, plasma, cerebrospinal fluid, aspiration cytology, among others, having as input obtained DNA from these different sources. In these examples, the advantage is to be able to make a determination from a less invasive kind of sample without having to rely on biopsy or surgery.

It can be applied in the surveillance of patients with bladder cancer via a DNA-based analysis result of a sample of urine. This application is based the fact that the mutation c.-124C>T and c.-146C> T are highly frequent in bladder cancer and can be detected in DNA derived from urine (Fig. 7) . For patients who are in clinical surveillance because they have 50-70% chance of recurrence, the possibility of detecting recurrence for urine testing has advantages over the conventional method based on cystoscopy to be completely non-invasive and more convenient for the patient.

The method of the present invention may also be applied to the detection of other types of cancer in which the Htert gene mutations are present and for other clinical purposes. One example is in the fine needle aspirates analysis of thyroid nodules in order to provide prognostic information useful in deciding the need for an ablative treatment or lymphadenectomy extension.

Another example of applying the present invention relates to early detection, diagnosis, prognosis, recurrence detection or prediction/monitoring response to therapy of tumors such as central nervous system cancers, bladder carcinomas, hepatocellular carcinomas, thyroid carcinomas, skin cancers, among others.

The present invention may also be useful for tumor tissue analysis or circulating DNA in the context of "liquid biopsy" for possible selection and treatment of candidate patients, response and prediction to treatment and monitoring, for example, with telomerase inhibitors for the Htert gene promoter.

### Brief Description of the Figures

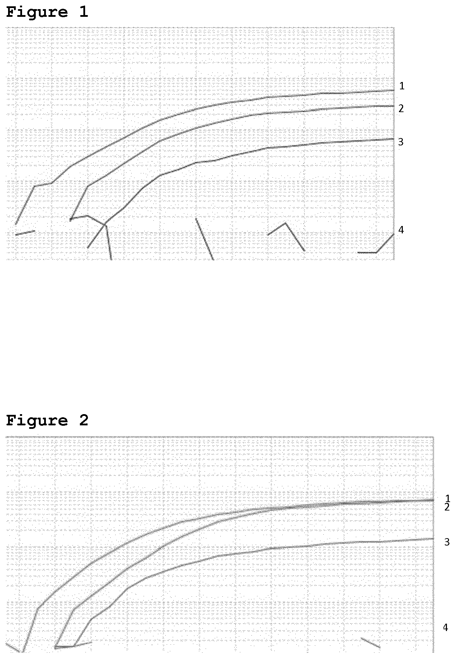

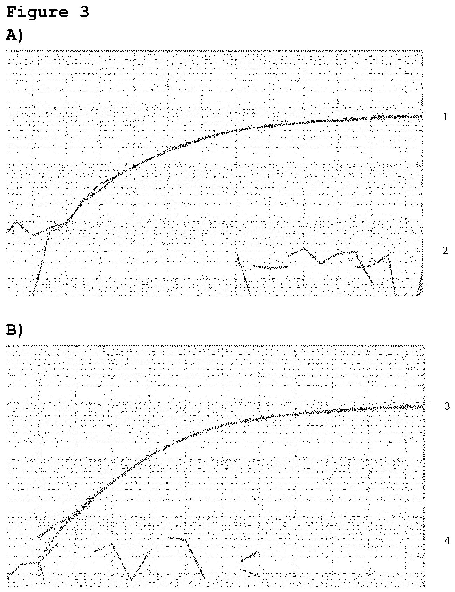

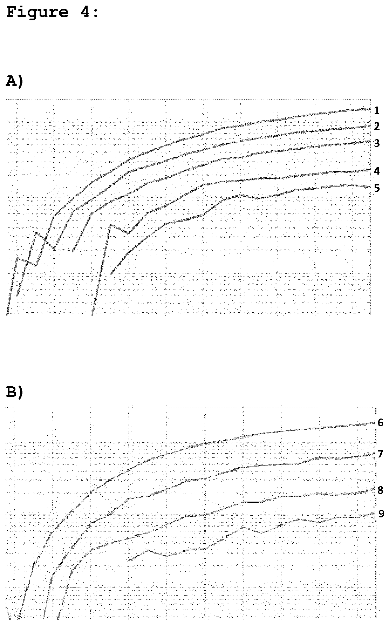

**Figure 1****:** Demonstration of the ability of the assay to detect mutations in the Htert gene promoter (*TERTp*) c.-124 C>T.
   In this figure it is presented that the assay is specific for the detection of alleles at position -124. The performance of the assay is confirmed by testing the c.-124 C>T mutation detection in heterozygous DNA samples for c.-124 C>T mutation (DNA HET124) and cases "wild-type" (DNA WT). The results demonstrate that the probe specific for the mutant allele (-124 Probe T) generates a signal in HET-124 DNA sample and not the WT DNA sample and the probe for allele "wild-type" (Probe-124 C) generates a signal amplification in both samples.
   1 - amplification curve obtained with the probe -124C (allele "wild-type") in DNA WT.
   2 - amplification curve obtained with the probe -124C (allele "wild-type") in HET-124 DNA.
   3 - amplification curve obtained with -124T probe (mutant allele) in HET-124 DNA.
   4 - amplification curve obtained with -124T probe (mutant allele) in WT DNA.
      X axis - amplification cycle
      Y axis - Fluorescence change
**Figure 2****:** Demonstration of the ability of the assay to detect mutations in *TERTp* c.-146 C>T
   In this figure it is presented that the assay is specific for the detection of alleles at position -146. The performance of the test in detecting the mutation c. -146 C> T is confirmed by the analysis of heterozygous DNA samples for c.-146 mutated C>T (DNA HET146) and cases wild-type (WT DNA). The results show that the probe specific for the mutant allele (-146 Probe T) generates a signal in HET-146 DNA sample and not in the WT DNA sample and the probe for allele "wild-type" (Probe-146 C) generates a signal amplification in both samples.
   1 - amplification curve obtained with the probe -146C (allele "wild-type") in DNA WT.
   2 - amplification curve obtained with -146T probe (mutant allele) in HET-146 DNA.
   3 - amplification curve obtained with the probe -146C (allele "wild-type") in HET-146 DNA.
   4 - amplification curve obtained with -146T probe (mutant allele) in WT DNA.
      X axis - amplification cycle
      Y axis - Fluorescence Change
**Figure 3****:** Evaluation of the analytical specificity of the tests:
   (A) specific detection of mutation *TERTp* c.-124 C>T
   (B) specific detection of mutation *TERTp* c.-146 C>T
   In this figure it is demonstrated that the assay does not have cross-reactivity between the tested mutations.
   Figure 3-A: DNA sample heterozygous for the mutation TERTp -146C>T is not amplified by the probe -124T and a single probe to emit fluorescence is in fact the probe -124C (corresponding to allele "wild-type" at position -124)
   FIG. 3-B: On the other hand, a DNA sample heterozygous for the mutation TERTp -124C>T is not amplified by the probe -146T and a single probe to emit fluorescence is in fact the probe -146C (corresponding to allele "Wild -Type "at position -146).
      1 - amplification curve obtained with the probe -124C (allele "wild-type") in HET-146 DNA.
      2 - amplification curve obtained with -124T probe (mutant allele) in HET-146 DNA.
      3 - amplification curve obtained with the probe -146C (allele "wild-type") in HET-124 DNA.
      4 - amplification curve obtained with -146T probe (mutant allele) in HET-124 DNA.
      X axis - amplification cycle
      Y axis - Fluorescence Change
**Figure 4****:** Evaluation of analytical sensitivity for detection of the - 124T allele (A) and -146T (B) under conditions that mimic DNA samples derived from biological fluids, in which there are small amounts of mutated alleles in a global context where the predominant allele is the "wild -type ".DNA samples were used with c.-124C>T mutation or samples with c.-146C>T mutation serially diluted in "wild-type" DNA samples.
   This figure reports the efficiency and sensitivity of the test in detecting mutated alleles -124T and -146T.
   1 - amplification curve obtained with probe -124T at 50% DNA MUT124 / DNAWT.
   2 - amplification curve obtained with the probe -124T 25% DNA MUT124 / DNAWT.
   3 - amplification curve obtained with the probe -124T 12.5% DNA MUT124 / DNAWT.
   4 - amplification curve obtained with -124T probe at 6.25% DNA MUT124 / DNAWT.
   5 - amplification curve obtained with -124T probe 3.125% DNA MUT124 / DNAWT.
   6 - Amplification curve obtained with probe -146T at 50% DNA MUT146 / DNAWT.
   7 - amplification curve obtained with the probe -146T 25% DNA MUT146 / DNAWT.
   8 - amplification curve obtained with the probe -146T 12.5% DNA MUT146 / DNAWT.
   9 - amplification curve obtained with -146T probe at 6.25% DNA MUT146 / DNAWT.
      X axis - amplification cycle
      Y axis - Fluorescence Change
**Figure 5****:** Potentiation of the analytical sensitivity in detecting-124T allele (A) and -146T (B).
   DNA samples were used with c.-124C>T mutation or samples with c.-146C>T mutation serially diluted in "wild-type" DNA samples in a proportion of 25% MUT DNA/DNA-WT.
   It can be observed that in a low concentration condition of the probe for allele "wild-type" (e.g. 250nM) the use of a higher concentration of the probe for the mutated alleles -124T and -146T (400-800-1600nM) results in a significant increase of the detection signal of the mutated alleles (Fig. 5 panels A and B, respectively).
   In panels C and D of this figure it can be seen that up to certain levels, the increase in the concentration of mutated probe does not result in nonspecific detection of allele "wild-type" as is evidenced by the absence of signal from probes -124T and -146T when using only DNA-WT as a sample.
   1 - amplification curve -124T probe (mutant) at a concentration of 400nm.
   2 - amplification curve -124C probe (wild-type) at a concentration of 250nM.
   3 - amplification curve -124T probe (mutant) at a concentration of 800nm.
   4 - amplification curve -124C probe (wild-type) at a concentration of 250nM.
   5 - amplification curve -124T probe (mutant) at a concentration of 1600nM.
   6 - Amplification curve -124C probe (wild-type) at a concentration of 250nM.
   7 - amplification curve -146T probe (mutant) at a concentration of 400nm.
   8 - Amplification curve -146C probe (wild-type) at a concentration of 250nM.
   9 - Amplification curve -146T probe (mutant) at a concentration of 800nm.
   10 - amplification curve -146C probe (wild-type) at a concentration of 250nM.
   11 - amplification curve -146T probe (mutant) at a concentration of 1600nM.
   12 - amplification curve -146C probe ("wild-type") at a concentration of 250nM.
   13 - amplification curve -124T probe (mutant) at a concentration of 1600nM.
   14 - amplification curve -124C probe ("wild-type") at a concentration of 250nM.
   15 - amplification curve -1246T probe (mutant) at a concentration of 400nm.
   16 - amplification curve -146C probe ("wild-type") at a concentration of 250nM.
      X axis - amplification cycle
      Y axis - Fluorescence Change
**Figure 6****:** Evaluation of analytical sensitivity for detection of the - 124T allele (A) and -146T (B) when increasing the concentration of the probe to the mutant allele and lowering the probe concentration used for allele "wild-type". This change in the relative composition of the probes results in increased analytical sensitivity.
   This figure presents the increased efficiency and sensitivity in the detection of mutated alleles -124T and -146T: the minimum detection values became 1.56% for -124T allele and 0.78% for -146T allele).
   1 - amplification curve obtained with the probe -124T 25% DNA MUT124 / DNAWT.
   2 - amplification curve obtained with the probe -124T 12.5% DNA MUT124 / DNAWT.
   3 - amplification curve obtained with -124T probe at 6.25% DNA MUT124 / DNAWT.
   4 - amplification curve obtained with -124T probe 3.125% DNA MUT124 / DNAWT.
   5 - amplification curve obtained with the probe -124T 1.56% DNA MUT124 / DNAWT.
   6 - Amplification curve obtained with probe -146T at 50% DNA MUT146 / DNAWT.
   7 - amplification curve obtained with the probe -146T 25% DNA MUT146 / DNAWT.
   8 - amplification curve obtained with the probe -146T 12.5% DNA MUT146 / DNAWT.
   9 - amplification curve obtained with -146T probe at 6.25% DNA MUT146 / DNAWT.
   10 - amplification curve obtained with -146T probe 3.125% DNA MUT146 / DNAWT.
   11 - amplification curve obtained with the probe -146T 1.56% DNA MUT124 / DNAWT.
   12 - amplification curve obtained with the probe -146T 0.78% DNA MUT146 / DNAWT.
      X axis - amplification cycle
      Y axis - Fluorescence Change
**Figure 7****:** Demonstration of the assay ability to detect *TERTp* mutations c.-124 C>T and c.-146 C>T in urine samples of patients with bladder cancer. In this figure you can be seen detection of c.-124 C>T mutation or c.-146 C>T mutation in urine samples. The results show that this type samples obtained from patients with bladder cancer, mutated alleles are detected for mutation -124 (A) or the - 146 mutation (B) as well as signals to the corresponding alleles "wild -type ".
   1 - amplification curve obtained with probe -124C (allele "wild-type") in DNA obtained from a urine sample of a patient with bladder cancer.
   2 - amplification curve obtained with -124T probe (mutant allele) in DNA obtained from a urine sample of a patient with bladder cancer.
   3 - amplification curve obtained with probe -146C (allele "wild-type") in DNA obtained from a urine sample of a patient with bladder cancer.
   4 - Amplification -146T curve obtained with probe (mutant allele) in DNA obtained from a urine sample of a patient with bladder cancer.
      X axis - amplification cycle
      Y axis - Fluorescence Change

### EXAMPLES

### Example 1 - Preparation of the sequences of primers and probes

Two primer sequences were prepared, SEQ ID NO: 1 and SEQ ID NO: 2, and 4 other sequences of the probes, SEQ ID NO: 3 SEQ ID NO: 6 as follows:
The sequences indicated above were prepared by nucleotide synthesis method using the solid phase phosphoramidite nucleoside. The synthesis was performed in packed columns with the functionalized solid support with the first base of the 3 'end of each oligonucleotide. The preparation of each oligonucleotide followed after a number of synthesis cycles, each consisting of four chemical reactions:
Unlock (detritylation); coupling; protection and oxidation. In each cycle were added step by step, the 5 'terminus of the growing chain, nucleotide residues corresponding to the desired sequence.

The concentration of probes was adjusted to values of 400 to 1600nM by dilution of a lyophilized preparation in double distilled water and double deionized water.

### Example 2 - Preparation of compositions PCR Reaction

Several PCR reactions compositions were prepared comprising the nucleotide according to sequences with SEQ ID NO: 1 to SEQ ID NO: 6 in different concentrations (for ex. 400nm, 800nm, 1600nM) and as described below. For comparison purposes, the solutions were also prepared with alternative concentration of the probe for allele "wild-type" (e.g., 250nM, 500nM).

### I - Compositions for mutation detection located at bases -124 upstream of the initiation codon ATG of the hTERT gene

### Inventive composition Ia:

- Solution reagent for Real-Time PCR " TaqMan ® Universal PCR Master Mix " at a concentration 1X;
- Oligonucleotide with the sequence: SEQ ID NO: 1, at a concentration of 900 nM;
- Oligonucleotide with the sequence: SEQ ID NO: 2, at a concentration of 900 nM;
- Oligonucleotide probe with the sequence: SEQ ID NO: 3, at a concentration of 250 nM, containing modifications such as the incorporation of the compound known as YAKIMA YELLOW ® and tetra- methyl-rhodamine (TAMRA) in the 5' and 3' terminals, respectively;
- Oligonucleotide probe with the sequence: SEQ ID NO: 4, at a concentration of 1600 nM, containing modifications such as the incorporation of the compound known as 6-carboxi-fluorescein (6-FAM) and tetra-methyl-rhodamine (TAMRA) in the 5' and 3' terminals, respectively;
- Water bi-distilled and bi-deionized.

### Inventive composition Ib:

This composition is prepared similarly to composition Ia but being the oligonucleotide probe concentration replaced with the one identified as: SEQ ID NO: 4, in a concentration of 800nm.

### Inventive composition Ic:

This composition is prepared similarly to composition Ia but being the oligonucleotide probe concentration replaced with the one identified as: SEQ ID NO: 4, in a concentration of 400nm.

### Comparative composition Id:

This composition is prepared similarly to composition Ia but being the oligonucleotide probe with the sequence: SEQ ID NO: 3, at a concentration of 500nM.

### II - Compositions for mutation detection located at -146 bases upstream of the initiation codon ATG of the hTERT gene

### Inventive composition IIa:

- Solution reagent for Real-Time PCR " TaqMan ® Universal PCR Master Mix " at a concentration 1X;
- Oligonucleotide with the sequence: SEQ ID NO: 1, at a concentration of 900 nM;
- Oligonucleotide with the sequence: SEQ ID NO: 2, at a concentration of 900 nM;
- Oligonucleotide probe with the sequence: SEQ ID NO: 5, at a concentration of 250 nM, containing modifications in the 5' and 3' terminals, respectively;
- Oligonucleotide probe with the sequence: SEQ ID NO: 6, at a concentration of 1600 nM, containing modifications in the 5' and 3' terminals, respectively;
- Water bi-distilled and bi-deionized.

The mentioned modifications at the 5' and 3' terminals sequences of the probes correspond to the addition of fluorophores (FAM, Yakima yellow) and quencher (TAMRA), etc. known to the expert.

### Inventive composition IIb:

This composition is prepared similarly to composition IIa however the oligonucleotide probe with the sequence: SEQ ID NO. 6, is present at a concentration of 800 nM.

### Inventive composition IIc:

This composition is prepared similarly to composition IIa however the oligonucleotide probe with the sequence: SEQ ID NO: 6, is present at a concentration of 400 nM.

### Comparative composition IId:

This composition is prepared similarly to composition Ia however the oligonucleotide probe with the sequence: SEQ ID NO: 5, is present at a concentration of 500 nM.

### III compositions for simultaneous detection of the mutations located at -124 and -146 bases upstream of the initiation codon ATG of the hTERT gene:

### Inventive composition IIIa:

- Solution reagent for Real-Time PCR " TaqMan ® Universal PCR Master Mix " at a concentration 1X;
- Oligonucleotide with the sequence: SEQ ID NO: 1, at a final concentration of 900 nM;
- Oligonucleotide with the sequence: SEQ ID NO: 2, at a final concentration of 900 nM;
- Oligonucleotide with the sequence: SEQ ID NO: 3, at a final concentration of 900 nM;
- Oligonucleotide probe with the sequence: SEQ ID NO: 4, at a concentration of 1600 nM, containing modifications in the 5' and 3' terminals, respectively;
- Oligonucleotide probe with the sequence: SEQ ID NO: 5, at a concentration of 250 nM, containing modifications in the 5' and 3' terminals, respectively;
- Oligonucleotide probe with the sequence: SEQ ID NO: 6, at a concentration of 1600 nM, containing modifications in the 5' and 3' terminals, respectively
- Water bi-distilled and bi-deionized.

### Inventive composition IIIb:

This composition is prepared similarly to composition IIIa, however the oligonucleotide probe with the sequence: SEQ ID NO: 4, is present in a concentration of 800 nM.

### Inventive composition IIIc:

This composition is prepared similarly to composition IIIa, however the oligonucleotide probe with the sequence: SEQ ID NO: 4, is present in a concentration of 400 nM.

### Inventive composition IIId:

This composition is prepared similarly to composition IIIa, however the oligonucleotide probe with the sequence: SEQ ID NO. 6, is present at a concentration of 800 nM.

### Inventive composition IIIe:

This composition is prepared similarly to composition IIIa, however the oligonucleotide probe with the sequence: SEQ ID NO: 6, is present at a concentration of 400 nM.

### Comparative composition IIIf:

This composition is prepared similarly to composition Ia, however the oligonucleotide probe with the sequence: SEQ ID NO: 3, is present at a concentration of 500 nM.

### Comparative composition IIIg:

This composition is prepared similarly to composition but being Ia probe oligonucleotide with the sequence: SEQ ID NO: 5, present at a concentration of 500nM.

### Example 3 - Preparation of compositions for detecting mutations

The compositions of the reaction mixtures for the detection of mutations c.-124 C>T and c.-146 C>T in the promoter of the gene hTERT were prepared with the addition of DNA from a biological test sample, approximately 100 ng of DNA, (it can be used lesser quantity, ranging from 1 ng to 500 ng) to the PCR compositions mentioned above, with the concentration of the probes with SEQ ID nr.3 and SEQ ID NO: 6, adjusted to values ranging from 400 to 1600nM, like described in the previous example. The compositions for detection were thus prepared with DNA samples containing the c.124 C>T mutation or samples containing the c.146 C>T mutation "serial diluted" in "wild-type" DNA samples.

Likewise, compositions for detection were prepared for comparative purposes, through the addition of DNA from the biological test sample to comparative solutions from the previous example.

### Example 4 - Method for the detection of the mutations c.-124 C>T and c.-146 C>T located in the promoter of the hTERT gene

For carrying out the method of the present invention there were prepared different compositions for detecting mutations as described in the previous example.

In this example it was used DNA from cell lines or from DNA tumor tissue, either fresh or Formalin Fixed Paraffin Embedded.

The compositions for detection from the previous example were subjected to amplification and hybridization processes in Real-Time PCR and Digital PCR. The compositions were also subjected to simultaneous assays (Multiplex) using in the same reaction probes for both mutations in analysis, being the probes labeled with different fluorophores. Among the most common fluorophores are known compounds known by Alexa Fluor FAM, TET, JOE, VIC, HEX, Cy3, ATTO 550, TAMRA, ROX, Cy5, Cy5.5.

For carrying out the method in Real Time PCR the ABI PRISM ® 7500 Fast machine marketed by Scientific ThermoFischer was used.

### Amplification and hybridization conditions:

1^{st} stage: 1 cycle of 10 minutes at 95 ° C
2^{nd} stage: 45 cycles, comprising 30 seconds at 92 ° C, 1 minute at 60 ° C, with temperature drop of 0.2 ° C per cycle starting from cycle 25
3^{rd} stage: 1 cycle of 1 minute at 57 ° C, with signal acquisition

### Example 5 - Method for the detection of the mutations c.-124 C>T and c.-146 C>T located in the promoter of the hTERT gene in a urine sample

Patient urine samples were obtained from bladder cancer patients in clinical or monitoring programs (cystoscopy) for tumor recurrence prevention. The urine was centrifuged at a force exceeding 1000 times the gravitational force (1000G) for a time period exceeding 5 minutes to pellet the epithelial cells from the bladder present in the urine. DNA was extracted from the obtained pelleted cells using common methods known in the art such as QIAamp® commercialized by Qiagen company or similar methods. Between 1 and 500 ng of the DNA obtained was added to the compositions for amplification and detection of mutations prepared as described in Examples 1, 2 and 3.

The compositions for detection from the example 3 were subjected to amplification and hybridization processes in Real-Time PCR and Digital PCR. The compositions were also subjected to simultaneous assays (Multiplex) using in the same reaction probes for both mutations in analysis, being the probes labeled with different fluorophores. Among the most common fluorophores are known compounds known by Alexa Fluor FAM, TET, JOE, VIC, HEX, Cy3, ATTO 550, TAMRA, ROX, Cy5, Cy5.5.

For carrying out the method in Real Time PCR the ABI PRISM ® 7500 Fast machine marketed by Scientific ThermoFischer was used.

### Amplification and hybridization conditions:

1^{st} stage: 1 cycle of 10 minutes at 95 ° C
2^{nd} stage: 45 cycles, comprising 30 seconds at 92 ° C, 1 minute at 60 ° C, with temperature drop of 0.2 ° C per cycle starting from cycle 25
3^{rd} stage: 1 cycle of 1 minute at 57 ° C, with signal acquisition

Using this method, the changes c.-124 C>T and c.-146 C>T in the hTERT gene promoter was detected in patients with bladder cancer both with low or high-grade, as well as in patients with recurrent bladder cancer (Figure 7).

### In conclusion:

The specificity of the probes was verified, i.e. if was evaluated if the probes for c.-124 C>T mutation (probes with SEQ ID NO: 3 and SEQ ID NO: 4) did not produce amplification of a heterozygous DNA sample for the c.-146 C>T alteration (DNA MUT 146) and vice versa (with the probes with SEQ ID NO: 5 and SEQ ID NO: 6) (Figure 3A and 3B). It was then verified that there are no phenomena of cross detection or false detection between the referred probes and the respective mutations.

In DNA samples derived from biological fluids, in which there are small amounts of mutated alleles in an environment dominated by "wild-type" alleles, the analytical sensitivity of -124T and -146T probes at a final concentration of 400nm at its respective mutation detection capability is maintained up to a maximum of 3.125% in the case of probe -124T and 6, 25% for the -146T probe (Figure 4a and b, respectively), which clearly shows the high efficacy of probes for the detection of mutations in high "wild-type" allele dilution conditions and therefore the ultra-sensitivity of the method of the present invention.

Under conditions that used 25% ADNMUT / ADNWT, it can be observed that in low concentration of the probe for the allele "wild-type" (e.g. 250nM) the use of a higher concentration of the probe for the mutated alleles -124T and -146T (400-800-1600nM) results in a significant increase of the detection signal of the mutated alleles (Fig. 5 panels a and B, respectively), which clearly indicates that by manipulating the concentration of the probes it is possible to adjust the sensitivity of the method of the present invention, for detecting the mutations in question.

To the increase the efficiency of detection of the mutated alleles - 124T and -146T, it corresponded a significant increase in the sensitivity of the analytical method of the invention, passing to minimum values of detection of 1.56% in the case of -124T allele and 0.78% in the case of -146T allele (Fig. 6A and B, respectively), which clearly indicates that by manipulating the concentration of the probes is possible to enhance the analytical sensitivity of the invention method for detecting the mutations in question, maintaining its specificity.

### SEQUENCE LISTING

### I - Primers

**SEQ ID** NO: **1:**
   5'-CCACGTGCGCAGCAGGAC-3'
**SEQ ID** NO: **2:**
   5'-CCGTCCTGCCCCTTCACCTT-3'

### II - Specific probes for the c.-124 C>T mutations in the promoter of the hTERT gene

**SEQ ID** NO: **3:**
   Yakima Yellow Dye-5'-AGGGCCCGGAGGGGGCT-3'-TAMRA
**SEQ ID** NO: **4:**
   FAM-5'-AGGGCCCGGAAGGGGCT-3'-TAMRA

### III - Specific Probes for the c.-146 C>T mutations in the promoter of the hTERT gene

**SEQ ID** NO: **5:**
   Yakima Yellow Dye-5'-CGGGGACCCGGGAGGGGT-3'-TAMRA
**SEQ ID** NO: **6:**
   FAM-5'-CGGGGACCCGGAAGGGGT-3'-TAMRA

### References:

1. Hayflick, L. and P.S. Moorhead, The serial cultivation of human diploid cell strains. Exp Cell Res, 1961. 25: p. 585-621.
2. Blackburn, E.H., Structure and function of telomeres. Nature, 1991. 350(6319): p. 569-73.
3. Greider, C.W., Telomerase is processive. Mol Cell Biol, 1991. 11(9): p. 4572-80.
4. Szostak, J.W. and E.H. Blackburn, Cloning yeast telomeres on linear plasmid vectors. Cell, 1982. 29(1): p. 245-55.
5. Gunes, C. and K.L. Rudolph, The role of telomeres in stem cells and cancer. Cell, 2013. 152(3):p.390-3
6. Murnane, J.P., Telomere dysfunction and chromosome instability. Mutat Res, 2012. 730(1-2): p. 28-36.
7. Cesare, A.J. and R.R. Reddel, Alternative lengthening of telomeres: models, mechanisms and implications. Nat Rev Genet, 2010. 11(5): p. 319-30.
8. Kim, N.W., et al., Specific association of human telomerase activity with immortal cells and cancer. Science, 1994. 266(5193): p. 2011-5.
9. Kyo, S., et al., Understanding and exploiting hTERT promoter regulation for diagnosis and treatment of human cancers. Cancer Sci, 2008. 99(8): p. 1528-38.
10. Aubert, G. and P.M. Lansdorp, Telomeres and aging. Physiol Rev, 2008. 88(2): p. 557-79.
11. Killela, P.J., et al., TERT promoter mutations occur frequently in gliomas and a subset of tumors derived from cells with low rates of self-renewal. Proc Natl Acad Sci U S A, 2013. 110(15): p. 6021-6.
12. Liu, X., et al., Highly prevalent TERT promoter mutations in aggressive thyroid cancers. Endocr Relat Cancer, 2013. 20(4): p. 603-10.
13. Nault, J.C., et al., High frequency of telomerase reverse-transcriptase promoter somatic mutations in hepatocellular carcinoma and preneoplastic lesions. Nat Commun, 2013. 4: p. 2218.
14. Vinagre, J., et al., Frequency of TERT promoter mutations in human cancers. Nat Commun, 2013. 4: p. 2185.

## Claims

1. A set of primers and probes for the detection of mutations c.-124 C>T and c.-146 C>T in the promoter of the gene hTERT **characterized by** comprising the primers with SEQ ID NO: 1 and SEQ ID NO: 2 and the probes with SEQ ID NO: 3, SEQ ID NO: 4, SEQ ID NO: 5 and SEQ ID NO: 6.

2. A composition for amplification and hybridization of DNA by PCR in an *in vitro* method of detection of mutations C.-124 C>T and c.-146 C>T in the promoter of the gene hTERT in a DNA sample **characterized by** comprising the set of primers and probes of claim 1 in addition to reagent solutions for PCR.

3. A composition according to claim 2, **characterized in that** at least one probe with SEQ ID NO: 3 to SEQ ID NO: 6 is present in the composition in a final concentration of 400nm to 1600nM.

4. A composition according to any of claims 2 to 3, **characterized in that** at least one probe with SEQ ID NO: 3 to SEQ ID NO: 6 is present in the composition in a final concentration of 800nm to 1600nM.

5. A composition according to any of claims 2 to 4, **characterized in that** the probes with SEQ ID NO: 3 and SEQ ID NO: 5 are present in the composition in the final concentration of 250nm to 500nM and **in that** the probes with SEQ ID NO: 4 and SEQ ID NO: 6 are present in the composition in a final concentration of 800nm to 1600nM.

6. A composition according to any of claims 2 to 5, **characterized in that** the probes with SEQ ID NO: 3 to SEQ ID NO: 6 are present in the composition in the following concentrations:
i) 250nM to 500nM of SEQ ID NO: 3, 400nm to 1600nM of SEQ ID NO: 4, 250nM to 500nM of SEQ ID NO: 5 and 400nm to 1600nM of SEQ ID NO: 6,
or
ii) 250nM to 500nM of SEQ ID NO: 3, 800nm to 1600nM of SEQ ID NO: 4, 250nM to 500nM of SEQ ID NO: 5 and 800nm to 1600nM of SEQ ID NO: 6.

7. A composition for use in an *in vitro* method of detection of mutations c.-124 C>T and c.-146 C>T in the promoter of the gene hTERT in a subject, **characterized by** comprising at least one composition according to any of claims 2 to 6 in addition to a human DNA sample isolated from a human biological sample.

8. A composition for use according to claim 7, **characterized in that** the DNA is present in the composition at a final concentration of less than 500ng, less than 100ng or less than 50ng and more than 1ng.

9. A kit for use in an *in vitro* method of detection of mutations c.-124 C>T and c.-146 C>T in the promoter of the gene hTERT in a subject, **characterized by** comprising at least one composition of claims 2 to 6.

10. A kit, for use according to claim 9, **characterized by** further comprising at least one DNA sample containing the mutation c.-124 C>T and c.-146 C>T in the promoter of the gene hTERT.

11. An *in vitro* method for the detection of the mutations c.-124 C>T and c.-146 C>T in the promoter of the gene hTERT in a DNA sample **characterized by** comprising the following steps:
a) Preparation of composition for detection as described in claims 7 to 8, by the promotion of a contact of the DNA sample extracted from a biological sample, to be tested for referred mutation, with a composition for amplification and hybridization by PCR as described in claims 2 to 6,
b) Amplification and hybridization of the DNA present in the sample by PCR reaction using compositions of claim 2 to 6,
c) Analysis of the amplification curves obtained in (b) and verification of the presence of exponential amplification of a fluorescence signal, which corresponds to the positivity for the presence of the c.-124 C>T mutation and c.-146 C>T mutation in the promoter of the gene hTERT in the sample under analysis.

12. The method of claim 11, wherein the DNA is isolated from abiological sample selected from urine, plasma, cerebrospinal fluid or aspiration cytology of a human being.

13. The method of any of claims 11 and 12, wherein the amount of DNA in the DNA sample of step (a) varies at a final concentration of less than 500ng, less than 100ng or less than 50ng and more than 1ng.

14. The method of any of claims 11 to 13, wherein in step b) the amplification conditions are as follows:
• 1^{st} stage: 1 cycle of 10 minutes at 95 ° C
• 2^{nd} stage: 45 cycles, comprising 30 seconds at 92 ° C, 1 minute at 60 ° C, with temperature drop of 0.2 ° C per cycle starting from cycle 25
• 3^{rd} stage: 1 cycle of 1 minute at 57 ° C, with signal acquisition.

## Patentansprüche

1. Ein Set von Primeren und Proben zur Erkennung von Mutationen c.-124 C>T und c.-146 C>T im Promotor des Gens hTERT **gekennzeichnet durch** Umfassen der Primere mit SEQ ID Nr. 1 und SEQ ID Nr. 2 und der Proben mit SEQ ID Nr. 3, SEQ ID Nr. 4, SEQ ID Nr. 5 und SEQ ID Nr. 6.

2. Eine Zusammensetzung zur Vervielfältigung und Hybridisierung von DNA durch PCR in eine *in* vitro-Methode zur Erkennung von Mutationen c.-124 C>T und c.-146 C>T im Promotor des Gens hTERT in einer DNA-Stichprobe **gekennzeichnet durch** Umfassen des Sets von Primeren und Proben gemäß Anspruch 1 zusätzlich zu PCR-Reagenzlösungen.

3. Eine Zusammensetzung gemäß Anspruch 2 **dadurch gekennzeichnet, dass** mindestens eine Probe mit SEQ ID Nr. 3 zu SEQ ID Nr. 6 in der Zusammensetzung in einer endgültigen Konzentration von 400nm bis 1600nM vorhanden ist.

4. Eine Zusammensetzung gemäß einer der Ansprüche 2 bis 3 **dadurch gekennzeichnet, dass** mindestens eine Probe mit SEQ ID Nr. 3 bis SEQ ID Nr. 6 in der Zusammensetzung in einer endgültigen Konzentration von 800nm bis 1600nM vorhanden ist.

5. Eine Zusammensetzung gemäß einer der Ansprüche 2 bis 4 **dadurch gekennzeichnet, dass** die Proben mit SEQ ID Nr. 3 und SEQ ID Nr. 5 in der Zusammensetzung in der endgültigen Konzentration von 250nm bis 500nM und die Proben mit SEQ ID Nr. 4 und SEQ ID Nr. 6 in der Zusammensetzung in einer endgültigen Konzentration von 800nm bis 1600nM vorhanden sind.

6. Eine Zusammensetzung gemäß einer der Ansprüche 2 bis 5 **dadurch gekennzeichnet, dass** die Proben mit SEQ ID Nr. 3 bis SEQ ID Nr. 6 in der Zusammensetzung in den folgenden Konzentrationen vorhanden sind:
i) 250nM bis 500nM von SEQ ID Nr. 3, 400nm bis 1600nM von SEQ ID Nr. 4, 250nM bis 500nM von SEQ ID Nr. 5 und 400nm bis 1600nM von SEQ ID Nr. 6,
oder
ii) 250nM bis 500nM von SEQ ID Nr. 3, 800nm bis 1600nM von SEQ ID Nr. 4, 250nM bis 500nM von SEQ ID Nr. 5 und 800nm bis 1600nM von SEQ ID Nr. 6.

7. Eine Zusammensetzung zur Anwendung in einer *in* vitro-Methode zur Erkennung von Mutationen c.-124 C>T und c.-146 C>T im Promotor des Gens hTERT in einem Probanden **gekennzeichnet durch** Umfassen mindestens einer Zusammensetzung gemäß einer der Ansprüche 2 bis 6 zusätzlich zu einer menschlichen DNA-Stichprobe isoliert von einer menschlichen biologischen Sitchprobe.

8. Eine Zusammensetzung zur Anwendung gemäß Anspruch 7 **dadurch gekennzeichnet, dass** das DNA in der Zusammensetzung bei einer endgültigen Konzentration von weniger als 500ng, weniger als 100ng oder weniger als 50ng und mehr als 1ng vorhanden ist.

9. Ein Kit zur Anwendung in einer *in* vitro-Methode zur Erkennung von Mutationen c.-124 C>T und c.-146 C>T im Promotor des Gens hTERT in einem Probanden **gekennzeichnet durch** mindestens eine Zusammensetzung der Ansprüche 2 bis 6.

10. Ein Kit zur Anwendung gemäß Anspruch 9 weiter umfassend mindestens eine DNA-Stichprobe beinhaltend die Mutation c.-124 C>T und c.-146 C>T im Promotor des Gens hTERT.

11. Eine *in* vitro-Methode zur Erkennung der Mutationen c.-1242 C>T und c.-146 C>T im Promotor des Gens hTERT in einer DNA-Stichprobe umfassend die folgenden Schritte:
a) Vorbereitung der Komposition zur Erkennung wie beschrieben in Ansprüche 7 bis 8, mittels Kontaktförderung der DNA-Stichprobe gewonnen aus einer biologischen Stichprobe, um für erwähnte Mutation getestet zu werden, mit einer Zusammensetzung zur Vervielfältigung und Hybridisierung mittels PCR wie beschrieben in Ansprüche 2 bis 6,
b) Vervielfältigung und Hybridisierung des DNA vorhanden in der Stichprobe mittels PCR-Reaktion durch Anwendung von Zusammensetzungen von Anspruch 2 bis 6,
c) Analyse der Vervielfältigungskurven gewonnen aus (b) und Überprüfung des Vorhandenseins einer exponentiellen Verstärkung eines Fluoreszenzsignals, was der Positivität der vorhandenen c.-124 C>T-Mutation und c.-146 C>T-Mutation im Promotor des Gens hTERT in der zu analysierenden Stichprobe entspricht.

12. Die Methode von Anspruch 11, wobei das DNA aus abiologischer Stichprobe isoliert wird, ausgewählt aus Urin, Plasma, Liquor oder Aspirationszytologie eines Menschen.

13. Die Methode von einer der Ansprüche 11 und 12, wobei sich die Menge an DNA in der DNA-Stichprobe von Schritt (a) bei einer endgültigen Konzentration von weniger als 500ng, weniger als 100ng oder weniger als 50ng und mehr als 1ng ändert.

14. Die Methode von einer der Ansprüche 11 bis 13, wobei die Vervielfältigungskonditionen in Schritt b) sind wie folgt:
• 1. Stadium: 1 Zyklus von 10 Minuten bei 95°C
• 2. Stadium: 45 Zyklen umfassend 30 Sekunden bei 92°C, 1 Minute bei 60°C, mit Temperatursturz von 0,2°C / Zyklus beginnend ab Zyklus 25.
• 3. Stadium: 1 Zyklus von 1 Minute bei 57°C mit Signalerfassung.

## Revendications

1. Ensemble d'amorces et de sondes pour la détection de mutations c.-124 C>T et c.-146 C>T dans le promoteur du gène hTERT, **caractérisé par le fait qu'**il comprend les amorces avec la SEQ ID NO: 1 et la SEQ ID NO: 2 et les sondes avec la SEQ ID NO: 3, la SEQ ID NO: 4, la SEQ ID NO: 5 et la SEQ ID NO: 6.

2. Composition pour une amplification et une hybridation de l'ADN par PCR dans le cadre d'une méthode *in vitro* de détection de mutations C.-124 C>T et c.-146 C>T dans le promoteur du gène hTERT dans un échantillon d'ADN **caractérisé par le fait qu'**il comprend un ensemble d'amorces et de sondes de la revendication 1, en plus de solutions réactives pour PCR.

3. Composition selon la revendication 2, **caractérisée par le fait qu'**au moins une amorce avec la SEQ ID NO: 3, jusqu'à SEQ ID NO: 6 est présente dans la composition dans une concentration finale de 400 nm à 1600 nM.

4. Composition selon la revendication 2 ou 3, **caractérisée par le fait qu'**au moins une sonde avec la SEQ ID NO: 3, jusqu'à la SEQ ID NO: 6 est présente dans la composition dans une concentration finale de 800nm à 1600nM.

5. Composition selon une quelconque revendication entre 2 et 4, **caractérisée par le fait que** les sondes avec la SEQ ID NO: 3 et la SEQ ID NO: 5 sont présentes dans la composition dans la concentration finale de 250nm à 500nM et **par le fait que** les sondes avec la SEQ ID NO: 4 et la SEQ ID NO: 6 sont présentes dans la composition dans une concentration finale de 800nm à 1600nM.

6. Composition selon une quelconque revendication entre 2 et 5, **caractérisée par le fait que** les sondes avec la SEQ ID NO: 3, jusqu'à la SEQ ID NO: 6 sont présentes dans la composition à des concentrations suivantes :
i) de 250nM à 500nM de la SEQ ID NO: 3, de 400nm à 1600nM de la SEQ ID NO: 4, de 250nM à 500nM de la SEQ ID NO: 5 et de 400nm à 1600nM de la SEQ ID NO: 6,
ou
ii)de 250nM à 500nM de la SEQ ID NO: 3, de 800nm à 1600nM de la SEQ ID NO: 4, de 250nM à 500nM de la SEQ ID NO: 5 et de 800nm à 1600nM de la SEQ ID NO: 6.

7. Composition pour une utilisation dans le cadre d'une méthode *in vitro* de détection de mutations c.-124 C>T et c.-146 C>T dans le promoteur du gène hTERT dans un sujet, **caractérisé par le fait qu'**il comprend au moins une composition selon une quelconque revendication entre 2 et 6 en plus d'un échantillon d'ADN humain isolé d'un échantillon biologique humain.

8. Composition pour une utilisation selon la revendication 7, **caractérisée par le fait que** l'ADN est présent dans la composition à une concentration finale de moins de 500ng, de moins de 100ng ou de moins de 50ng et de plus de 1ng.

9. Kit d'utilisation dans le cadre d'une méthode *in vitro* de détection de mutations c.-124 C>T et c.-146 C>T dans le promoteur du gène hTERT dans un sujet, **caractérisé par le fait qu'**il comprend au moins une composition selon les revendications entre 2 et 6.

10. Kit d'utilisation selon la revendication 9, **caractérisé par le fait qu'**il comprend au moins un échantillon d'ADN contenant la mutation c.124 C>T et c.-146 C>T dans le promoteur du gène hTERT.

11. Méthode *in vitro* pour la détection des mutations c.-124 C>T et c.-146 C>T dans le promoteur du gène hTERT dans un échantillon d'ADN, **caractérisée par le fait qu'**elle comprend les étapes suivantes :
a) Préparation de la composition pour la détection telle que décrite dans les revendications 7 et 8, par la promotion d'un contact de l'échantillon d'ADN extrait d'un échantillon biologique à être testé pour la mutation visée, avec une composition pour l'amplification et l'hybridation par PCR, tel que décrit dans les revendications 2 à 6,
b) Amplification et hybridation de l'ADN présent dans l'échantillon par réaction PCR en utilisant des compositions des revendications 2 à 6,
c) Analyse des courbes d'amplification obtenues sur (b) et vérification de la présence de l'amplification exponentielle d'un signal qui correspond à la positivité pour la présence de la mutation c.-124 C>T et de la mutation c.-146 C>T dans le promoteur du gène hTERT dans l'échantillon sous analyse.

12. Méthode de la revendication 11, où l'ADN est isolé de l'échantillon abiologique sélectionné de l'urine, du plasma, du fluide cérébrospinal ou de la cytoponction d'un être humain.

13. Méthode de la revendication 11 ou 12, où la quantité d'ADN dans l'échantillon d'ADN de l'étape (a) varie à une concentration finale de moins de 500ng, moins de 100ng ou moins de 50ng et de plus de 1ng.

14. Méthode d'une quelconque revendication entre 11 et 13, où, à l'étape b), les conditions d'amplification sont les suivantes
• le phase: 1er cycle de 10 minutes à 95°C
• 2e phase: 45 cycles, comprenant 30 secondes à 92 °C, 1 minute à 60 °C avec une baisse de température de 0,2 °C par cycle en commençant par le cycle 25
• 3e étape: 1 cycle de 1 minute à 57 °C, avec acquisition de signal.


### SEQUENCE LISTING

<110> IPATIMUP (INSTITUTO DE PATOLOGIA E IMUNOLOGIA MOLECULAR DA UNIVERSIDADE DO PORTO)
<120> MÉTODO, SEQUÊNCIAS, COMPOSIÇÕES E ESTOJO PARA DETECÇÃO DE MUTAÇÕES NO PROMOTOR DO GENE Htert
<130> 11670IB
<140> PT108419
   <141> 2015-04-30
<160> 6
<170> PatentIn version 3.5
<210> 1
   <211> 0
   <212> DNA
   <213> Homo sapiens
<400> 1
   000
<210> 2
   <211> 0
   <212> DNA
   <213> Homo sapiens
<400> 2
   000
<210> 3
   <211> 0
   <212> DNA
   <213> Homo sapiens
<400> 3
   000
<210> 4
   <211> 0
   <212> DNA
   <213> Homo sapiens
<400> 4
   000
<210> 5
   <211> 0
   <212> DNA
   <213> Homo sapiens
<400> 5
   000
<210> 6
   <211> 0
   <212> DNA
   <213> Homo sapiens
<400> 6
   000